# EUROPEAN PATENT APPLICATION

(11) **EP 1 447 629 A1**
(43) Date of publication of application: **18.08.2004**
(21) Application number: 03257213.3
(22) Date of filing: 14.11.2003
(51) Int. Cl.: F24F 7/007, F04D 25/08, A61L 9/12

(54) **Air circulating device**

(30) Priority: 12.02.2003 KR 2003008769
(71) Applicant: SAMSUNG ELECTRONICS CO., LTD., Suwon-City, Kyungki-do (KR)
(72) Inventor: Kim, Jung Ho, Suwon-City Kyungki-Do (KR); Chai, Kyung Ho, Suwon-City Kyungki-Do (KR); Lee, Jai Kwon, Suwon-City Kyungki-Do (KR)
(74) Representative: Brandon, Paul Laurence

(57) **Abstract**

An air circulating device including a housing (10) which contains a blowing fan (15) and a motor (16). The housing (10) includes an air inlet port (13) and an air outlet port (14), with a plug (20) provided at a surface of the housing (10) to be connected to a power source. The blowing fan (15) is set in the housing (10), as is the motor (16) which drives the blowing fan (15).

## Description

The present invention relates, in general, to air circulating devices. More particularly, but not exclusively, the present invention relates to an air circulating device which is designed such that the body thereof is directly connected to an electrical wall socket to circulate indoor air and ventilate a room.

Recently, air conditioners and cleaners have been widely used in homes-to-control the temperature, humidity, and sanitation of a room, thus keeping the room comfortable. The air conditioners function to cool or heat indoor air. The air cleaners function to clean indoor air. Such devices forcibly circulate indoor air by their blowing means, thus conditioning and cleaning indoor air.

However, an air conditioner or air cleaner is installed at a fixed place in a room. Thus, in the case where the room is large, air around the air conditioner or the air cleaner is smoothly circulated, thus accomplishing a high air conditioning effect or a high air cleaning effect. But air in a place distant from the air conditioner or the air cleaner is not smoothly circulated, so an air conditioning effect or an air cleaning effect is somewhat poor. Thus, it takes a long time to uniformly condition or clean indoor air.

Accordingly, it is an aim of preferred embodiments of the present invention to provide an air circulating device which allows indoor air to be smoothly circulated and allows a room to be effectively ventilated. Another aim of preferred embodiments of the present invention is that the air circulating device be small in size, thus being easy to carry and store.

It is another aim of preferred embodiments of the present invention to provide an air circulating device which is designed such that it is connected to and held by a socket, and is designed to allow a plug of another electrical device to be connected to a portion of the air circulating device.

According to the present invention in a first aspect, there is provided an air circulating device, comprising: a housing having an air inlet port and an air outlet port, with a plug provided at a surface of the housing to be connected to a power source, a blowing fan set in the housing, and a motor to drive the blowing fan.

The air inlet port may be provided at a front of the housing, and the air outlet port may be provided on an outer circumferential surface of the housing.

Also, the housing may include a front casing provided with the air inlet port, and a rear casing assembled with the front casing and provided on an outer circumferential surface thereof with the air outlet port.

The air circulating device may also include a mode conversion switch to change a rotating direction of the blowing fan by converting power applied to the motor, thus allowing the air inlet port to serve as an air outlet port while allowing the air outlet port to serve as an air inlet port.

Further, the air circulating device may include a timer mounted to a predetermined portion of the housing so as to control an operating time of the blowing fan.

A socket may be provided at a surface of the housing, thus allowing a plug of another electrical device to be connected to the socket.

An air circulating device comprising: a housing having an air inlet port and an air outlet port; a plug provided at a surface of the housing to be connected to a power source; and a socket provided at a surface of the housing, wherein a plug of another electrical device connects to the socket and is powered by the same source as the air circulating device.

An air circulating device comprising: a housing having an air inlet port and an air outlet port; and a plug provided at a surface of the housing to be connected to a power source, wherein the air circulating device is structurally supported by the plug when connected to a wall outlet.

Further features of the present invention are set out in the appended claims set.

The present invention will become more apparent and more readily appreciated from the following description of the preferred embodiments, by way of example only, taken in conjunction with the accompanying drawings of which:
Figure 1 is a perspective view of an air circulating device, according to an embodiment of the present invention;
Figure 2 is an exploded perspective view illustrating an interior of the air circulating device, according to an embodiment of the present invention;
Figure 3 is a perspective view of an air circulating device, according to another embodiment of the present invention;
Figure 4 is a perspective view illustrating an example of an operation of the air circulating device of Figure 1; and
Figure 5 is a perspective view illustrating another example of an operation of the air circulating device of Figure 1.

Reference will now be made in detail to the present preferred embodiments of the present invention, examples of which are illustrated in the accompanying drawings, wherein like reference numerals refer to like elements throughout. The embodiments are described below in order to explain the present invention by referring to the figures.

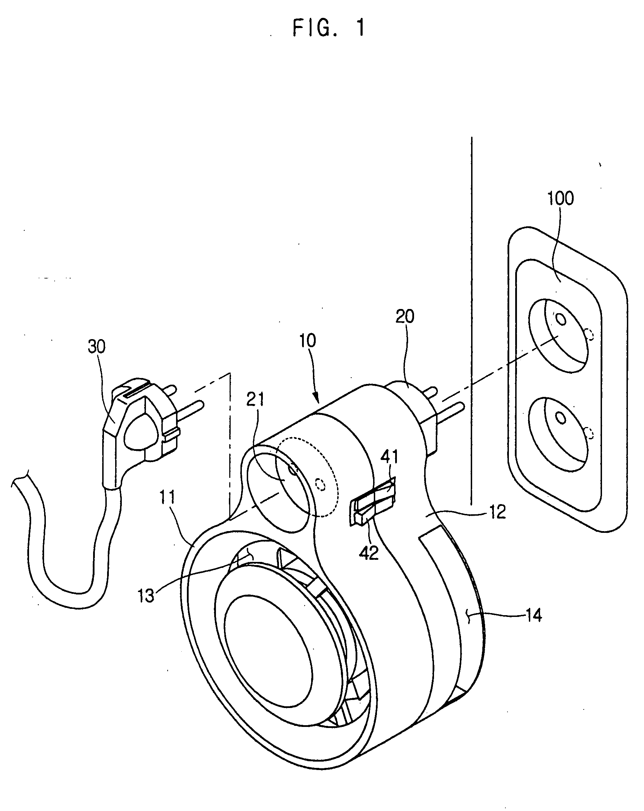

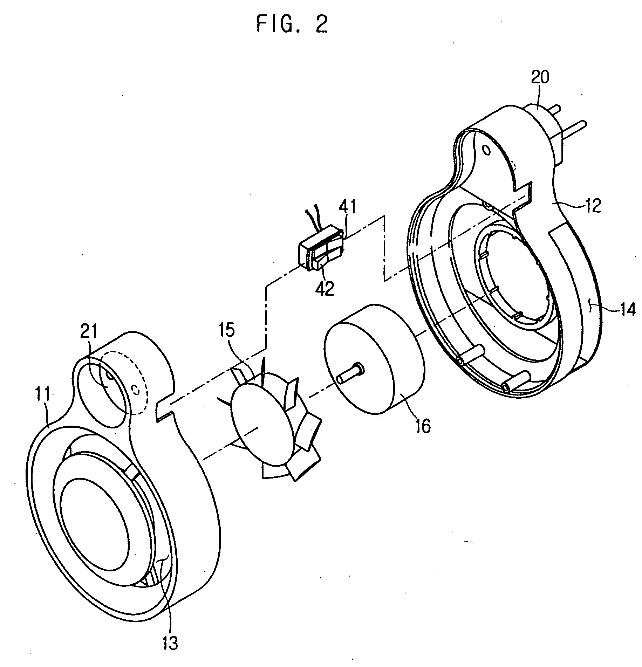

As illustrated in Figures 1 and 2, an air circulating device according to an embodiment of the present invention includes a housing 10 which has a front casing 11 and a rear casing 12. The front and rear casings 11 and 12 are removably assembled with each other. An air inlet port 13 is provided at a front of the front casing 11 of the housing 10 to suck indoor air into the housing 10. An air outlet port 14 is provided on an outer circumferential surface of the rear casing 12 of the housing 10 to discharge air to a room.

Further, an axial blowing fan 15 and a motor 16 are set in the housing 10. The blowing fan 15 forcibly circulates indoor air so that indoor air is sucked into the air inlet port 13 of the housing 10, and is discharged through the air outlet port 14 to the room. The motor 16 drives the blowing fan 15. In this case, the motor 16 comprises a reversible motor which is able to rotate in alternating directions. The motor 16 is mounted at a surface thereof to a center of an inner surface of the rear casing 12.

As illustrated in Figure 1, a plug 20 is integrally provided at an upper portion of the rear casing 12 of the housing 10 to be connected to a wall-mounted socket 100. Such a construction allows the housing 10 to be held by the wall-mounted socket 100 while allowing power to be applied to the motor 16, when the plug 20 is connected to the socket 100.

A built-in electrical socket 21 is integrally provided at a surface of the housing 10 opposite to the surface having the plug 20, thus allowing a plug 30 of another electrical device to be connected to the socket 21. Thus, although the air circulating device is connected to the wall-mounted socket 100 and covers the socket 100, it is possible to connect the plug 30 of another electrical device to the built-in socket 21 which is provided at the upper portion on the front of the housing 10.

Further, a power switch 41 and a mode conversion switch 42 are provided at a predetermined portion of the housing 10. The power switch 41 functions to control the operation of the motor 1 6. The mode conversion switch 42 functions to change the rotating direction of the blowing fan 15 by converting power applied to the motor 16. When the rotating direction of the blowing fan 15 is changed by the mode conversion switch 42, the air blowing direction is changed, so the air inlet port 13 serves as an air outlet port, while the air outlet port 14 serves as an air inlet port.

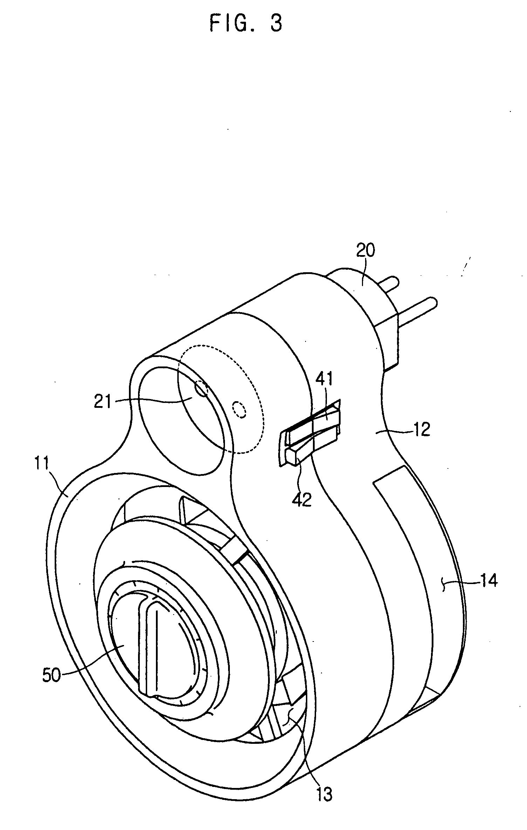

Figure 3 illustrates an air circulating device according to another embodiment of the present invention. According to the air circulating device illustrated in Figure 3, a timer 50 is mounted to a predetermined portion of the front casing 11 so as to control an operating time of the blowing fan 15. The timer 50 allows a user to preset the operating time, thus making the air circulating device more convenient to use.

Examples of use of the air circulating device according to preferred embodiments of the present invention will be described in the following.

The air circulating device of preferred embodiments of the present invention may serve as an auxiliary air circulating device in a room where an air conditioner or an air cleaner is installed, thus enhancing circulating efficiency of indoor air. Alternatively, the air circulating device may be installed in a room without the air conditioner or the air cleaner, thus circulating indoor air and ventilating the room.

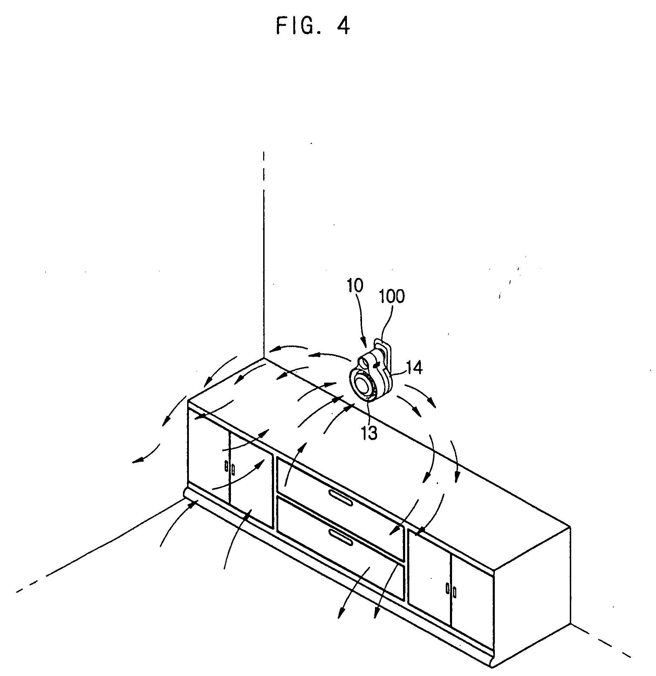

When one desires to use the air circulating device, as illustrated in Figure 4, the plug 20 of the housing 10 is connected to the wall-mounted socket 100. Next, the power switch 41 is turned on to operate the air circulating device. At this time, the blowing fan 15 set in the housing 10 is operated, so indoor air is sucked into the air inlet port 13 of the housing 10 and is discharged through the air outlet port 14 to the room in a radial direction, thus circulating the indoor air. When one desires to change the air blowing direction, the mode conversion switch 42 is manipulated so that the blowing fan 15 is rotated in an opposite direction. Further, it is possible to preset a desired operating time using the timer 50.

Meanwhile, when one desires to use another electrical device during an operation of the air circulating device, as illustrated in Figure 1, a user has only to connect the plug 30 of another electrical device to the built-in socket 21 which is integrated with the housing 10 of the air circulating device.

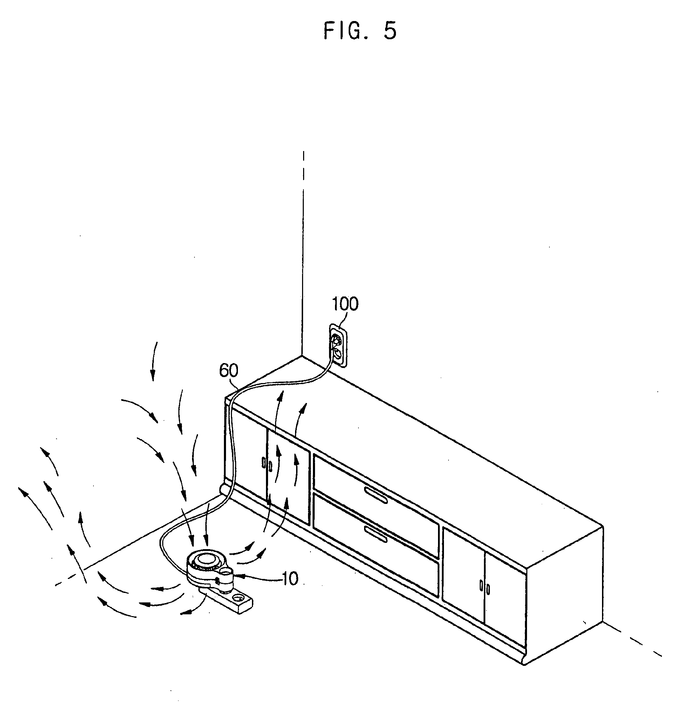

As illustrated in Figure 5, when one desires to place the air circulating device at a center or other desired position of a room to enhance air circulation as well as a ventilating effect, a user has only to use an extension cable 60.

In another embodiment of the present invention the housing 10 of the air circulating device is equipped with a unit to support a scented material, such as an oil or other liquid or solid air freshener, which will add a pleasant fragrance to the indoor air that is being circulated. In normal operation of the air circulating device, as shown in Figures 4 and 5, the scented material would be mingled with the air before it leaves the air outlet port 14, producing a pleasing smell with the circulated indoor air.

As is apparent from the above description, preferred embodiments of the present invention provide an air circulating device, which serves to circulate indoor air as an auxiliary device of an air conditioner or an air cleaner, and which also serves as an independent air circulating device, thus keeping a comfortable circulation of indoor air inside a room.

Preferred embodiments of the present invention provide an air circulating device, which is small in size, thus being easily connected to a socket, and thus being easy to carry and store.

Furthermore, preferred embodiments of the present invention provide an air circulating device which allows a plug of another electrical device to be connected to a socket provided at an upper portion of a housing.

Although a few embodiments of the present invention have been shown and described, it would be appreciated by those skilled in the art that changes may be made in these embodiments without departing from the principles and spirit of the invention, the scope of which is defined in the claims and their equivalents.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. An air circulating device, comprising:
a housing (10) having an air inlet port (13) and an air outlet port (14), with a plug (20) provided at a surface of the housing (10) to be connected to a power source (100);
a blowing fan (15) set in the housing (10); and
a motor (16) to drive the blowing fan (15).

2. The air circulating device according to claim 1, wherein the air inlet port (13) is provided at a front of the housing (10), and the air outlet port (14) is provided on an outer circumferential surface of the housing (10).

3. The air circulating device according to claim 1 or claim 2, wherein the housing (10) comprises:
a front casing (11) provided with the air inlet port (13); and
a rear casing (12) assembled with the front casing (11), wherein the air outlet port (14) is provided on an outer circumferential surface of the rear casing (12).

4. The air circulating device according to any preceding claim, further comprising:
a power switch (41); and
a mode conversion switch (42), wherein the power switch (41) and the mode conversion switch (42) are provided at a predetermined portion of the housing (10) .

5. The air circulating device according to claim 4, further comprising a reversible motor (16), wherein the mode conversion switch (42) changes a rotating direction of the blowing fan (15), thus allowing the air inlet port (13) to serve as an air outlet port (14) while allowing the air outlet port (14) to serve as an air inlet port (13).

6. The air circulating device according to any preceding claim, further comprising a timer (50) mounted to a predetermined portion of the housing (10), wherein the timer (50) controls an operating time of the blowing fan (15).

7. The air circulating device according to any preceding claim, further comprising a socket (21) provided at a surface of the housing (10), thus allowing a plug (30) of another electrical device to be connected to the socket (21).

8. The air circulating device of claim 7, wherein the socket (21) is provided opposite to the surface of the housing (10) having the plug (20).

9. The air circulating device according to any preceding claim, wherein the air circulating device is structurally supported by the plug (20) when connected to a wall outlet.

10. The air circulating device according to any preceding claim, wherein the housing (10) further comprises a unit to support a scented material, to affect the olfactory quality of the circulated air.

11. An air circulating device comprising:
a housing (10) having an air inlet port (13) and an air outlet port (14);
a plug (20) provided at a surface of the housing (10) to be connected to a power source; and
a socket (21) provided at a surface of the housing (10), wherein a plug (30) of another electrical device connects to the socket (21) and is powered by the same source as the air circulating device.

12. An air circulating device comprising:
a housing (10) having an air inlet port (13) and an air outlet port (14); and
a plug (20) provided at a surface of the housing (10) to be connected to a power source, wherein the air circulating device is structurally supported by the plug (20) when connected to a wall outlet (100).
